# EUROPÄISCHE PATENTANMELDUNG

(11) **EP 4 400 575 A1**
(43) Veröffentlichungstag der Anmeldung: **17.07.2024**
(21) Anmeldenummer: 23151440.7
(22) Anmeldetag: 13.01.2023
(51) Int. Cl.: C12M 1/107, C12M 1/04, C12M 1/00, C12M 1/34, C12M 1/33

(54) **ANLAGE ZUR VORBEHANDLUNG FESTER EINSATZSTOFFE ZUR ERZEUGUNG VON BIOGAS, BIOGASANLAGE UND VERFAHREN ZUR ERZEUGUNG VON BIOGAS**

(71) Anmelder: MTM Anlagenbau GmbH, 91611 Lehrberg (DE)
(72) Erfinder: Rabe, Petra, 15713 Königs Wusterhausen (DE); Spitzner, Ulrich, 91611 Lehrberg (DE)
(74) Vertreter: Gulde & Partner

(57) **Zusammenfassung**

Die Erfindung betrifft eine Anlage zur Vorbehandlung von festen Substraten in Biogasanlagen, und eine Steuerungseinheit, die ausgebildet ist, eine zuzuführende Menge an Flüssigkeit aus dem Vergleich einer aktuellen Stromaufnahme des Rührwerks mit einer vorgegebenen Stromaufnahme des Rührwerks zu steuern, die Temperatur in der Turbomaische zu regeln; und die zuzuführende Luftmenge sowie die Dauer der belüfteten Intervalle und unbelüfteten Intervalle aus der Bewertung der Gaskonzentrationen in der Abluft, insbesondere von Sauerstoff und Kohlendioxid, sowie dem Gärsäurespektrum in der Maischeflüssigkeit zu steuern. Ferner umfasst die Erfindung eine Biogasanlage und ein Verfahren zu Gewinnung von Biogas.

## Beschreibung

Die Erfindung betrifft eine Anlage, Turbomaische genannt, zur Vorbehandlung fester Einsatzstoffe, auch Substrate genannt, zur Erzeugung von Biogas. Ferner wird eine Biogasanlage als auch ein Verfahren zur Erzeugung von Biogas aus festen Substraten beschrieben.

### Hintergrund der Erfindung

Biogas als alternative Energiequelle gewinnt vor dem Hintergrund der schwindenden Ressourcen nichterneuerbarer Energieträger zunehmend an Bedeutung. Biogas ist ein brennbares Gas, das durch Vergärung von Biomasse entsteht. Es wird in Biogasanlagen hergestellt, wozu sowohl organische Abfälle als auch nachwachsende Rohstoffe als Ausgangsstoffe verwendet werden können. Herkömmlicherweise wird bei der Biogaserzeugung vorwiegend auf Maissilage als Basis abgestellt.

Anlagenbetreiber stellen aber zunehmend auch auf die Nutzung landwirtschaftlicher Reststoffe, wie Stroh und Mist, ab.

Biogasanlagen müssen hierzu sowohl auf den Betrieb mit diesen Reststoffen umgerüstet werden, als auch, an die flexible Vermarktung des Biogases in Form von Wärme und Strom oder Biomethan angepasst werden. Es gelangen zunehmend tierische Exkremente als Einsatzstoffe für Biogasanlagen in den Fokus. Mist von Rindern, Pferden, Schweinen, Hühnern und anderen Tieren ist in dieser Hinsicht besonders geeignet, da er aufgrund des Strohs aus der Einstreu einen hohen Energiegehalt besitzt.

Der Einsatz dieser Substrate in Biogasanlagen, die ursprünglich für die Nutzung von Mais geplant waren, führt allerdings zu erheblichen technologischen und verfahrenstechnischen Problemen.

Zum einen schwankt der Strohanteil und damit der Energiegehalt der Substrate stark. Mist rottet beispielsweise schnell und verliert aufgrund dessen während der Lagerung in kurzer Zeit einen erheblichen Teil seines Energiegehaltes. Um die benötigte Biogasmenge zuverlässig zu produzieren, muss die Menge an Einsatzstoffen also ständig angepasst werden.

Ferner erfordern die jahreszeitlichen Schwankungen des Energiebedarfs den Prozess an, die damit verbundenen starken Schwankungen der Einsatzmengen anpassen zu können.

Die Substrate sollen in der Turbomaische möglichst dick mit Flüssigkeit angemischt werden, denn Überschussflüssigkeit würde den Methanfermenter unnötig belasten. So können Kosten für die Ausbringung von flüssigem Gärrest vermieden werden und der Sauerstoffeintrag in die Flüssigkeit verbessert werden. Begrenzt wird die Verringerung der Flüssigkeitsmenge allerdings durch die Rühr- und Pumpfähigkeit der Maischeflüssigkeit.

Ferner soll es möglich sein, auch reines Stroh in der Biogasanlage zu vergären, ohne dass es zur Schwimmschichtbildung im Fermenter kommt.

Hierbei ist zu beachten, dass in der Turbomaische unter anderem aerobe biologische Prozesse erfolgen, die vorzugsweise im Temperaturbereich zwischen 30 °C und 38 °C ablaufen und eine positive Wärmetönung besitzen, welche eine Selbsterwärmung des Behälters um mehrere Grad Celsius bewirkt. Andererseits besitzt insbesondere Mist schon nach sehr kurzer Lagerzeit häufig Temperaturen von bis zu 60 °C. Die zugeführte Flüssigkeit zum Anmischen besteht häufig aus abgepresstem Gärrest, der eine Temperatur von bis zu 45 °C aufweisen kann. Das erfordert eine Temperierung des Behälters, der sowohl im Winter geheizt als auch gegebenenfalls im Sommer abgekühlt werden muss.

Der Mist enthält neben Sand und Steinen häufig erhebliche Mengen an Störstoffen, wie Metallteile, Holzstücke, Schnüre, Ausrüstungsteile. Diese müssen aus dem Prozess entfernt werden, bevor sie in eine Pumpe gelangen. Die normalerweise vor Pumpen angeordneten Steinfänge sind kaum in der Lage diese Störstoffe nach Art und Menge von der Pumpe fernzuhalten.

Der Erfindung liegt nun die Aufgabe zugrunde, eine Anlage für ein verbessertes Verfahren zur Erzeugung von Biogas bereitzustellen, welches die noch bestehenden Probleme adressiert. Insbesondere soll das Verfahren auf die Behebung der Schwierigkeiten abstellen, die durch die Schwankungen der Substratqualität hervorgerufen werden, wie diese insbesondere bei der Verwendung von Reststoffen als Substrat auftreten.

### Zusammenfassung der Erfindung

Diese Aufgabe wird mit einer Anlage zur Vorbehandlung von festen Substraten in Biogasanlagen, genannt Turbomaische, einer Biogasanlage sowie durch ein dazugehöriges Verfahren zur Biogasgewinnung gelöst.

Anlage zur Vorbehandlung von festen Substraten in Biogasanlagen, umfassend:
einen Behälter dessen Höhe zu Durchmesser-Verhältnis mindestens 1 : 3, also 0,33, beträgt, ein mindestens zweistufiges Großflügel-Propellerrührwerk zum Einmischen der festen Substrate; und mindestens einen Belüftungsring umfassend wenigstens ein Belüftungsorgan, die durch einen Verdichter mit Luft versorgt werden; sowie
eine Steuerungseinheit, die ausgebildet ist:
   eine zuzuführende Menge an Flüssigkeit aus dem Vergleich einer aktuellen Stromaufnahme (Iᵢₛₜ) des Rührwerks mit einer vorgegebenen Stromaufnahme (Iₛₒₗₗ) des Rührwerks zu steuern; die gewünschte Temperatur in der Turbomaische durch Heizen oder Kühlen zu regeln und die erforderliche zuzuführende Luftmenge und Intervalldauer der belüfteten und unbelüfteten Intervalle aus den Abluftwerten, und/oder dem Säurespektrum in der Turbomaische zu steuern.

Damit ist die erfindungsgemäße Anlage in der Lage, trotz unterschiedlicher zur Verfügung stehender Mengen und Qualitäten der verschiedenartigen, faserhaltigen und störstoffbelasteten Einsatzstoffen eine flexibel an den Energiebedarf angepasste Biogasmenge bereitzustellen.

Insbesondere wird den Besonderheiten der Viskosität der faserhaltigen Einsatzstoffe Rechnung getragen. Wegen der unterschiedlichen Qualitäten der verschiedenartigen, faserhaltigen und störstoffbelasteten Einsatzstoffen liegt ein besonderes Augenmerk auf der Strukturviskosität der Mischung. Als Strukturviskosität, auch Scherentzähung genannt, wird die Eigenschaft eines Fluids, bei hohen Scherkräften eine abnehmende Viskosität zu zeigen, bezeichnet. Je stärker also die Scherung ist, die auf das Fluid wirkt, desto weniger viskos, zähflüssig, wird es.

Im vorliegenden Fall liegt vor dem Hintergrund der Art der festen in Flüssigkeit dispergierten Substrate eine strukturviskose Flüssigkeit vor. Herkömmliche Viskositätsmessungen (beispielsweise durch Probeentnahme und Messung mit einem Viskosimeter) sind für die vorliegenden strukturviskosen Flüssigkeiten nicht geeignet. Die erfindungsgemäße Anlage trägt dem dadurch Rechnung, dass die Stromaufnahme des Rührers als Leitgröße für die Flüssigkeitszufuhr genutzt wird, um hierdurch die notwendige Zufuhr von Flüssigkeit zum Erreichen des höchstmöglichen Anteils an festen Substraten zu realisieren.

Die Messung der Viskosität strukturviskoser Flüssigkeiten unter bestimmten Scherbedingungen direkt im Behälter ist messtechnisch praktisch nicht zu realisieren. Sie ist auch nicht zielführend. Ziel ist es, dem Behälter so wenig wie möglich Flüssigkeit zuzuführen um einerseits so viel wie möglich feste Substrate einzubringen und für den biologischen Prozess im nachfolgenden Fermentationsprozess eine möglichst lange hydraulische Verweilzeit zu sichern. Andererseits verhindert eine hohe Viskosität die Koaleszenz der an den Belüftern gebildeten kleinen Luftblasen und sichert durch die stark vergrößerte Blasenoberfläche einen besseren Sauerstoffeintrag in die Flüssigkeit und infolgedessen die Verringerung des insgesamt benötigten Luftbedarfs im Behälter.

Die zuzuführende Menge an Flüssigkeit aus dem Vergleich einer aktuellen Stromaufnahme (Iᵢₛₜ) des Rührwerks mit einer vorgegebenen Stromaufnahme (Iₛₒₗₗ) des Rührwerks zu steuern hat sich damit als vorteilhaftes Verfahren zu Flüssigkeitsdosierung erwiesen.

Ferner findet gemäß einer bevorzugten Ausführungsform eine Steuerung der zuzuführenden Menge an festen Substraten aus dem Vergleich eines aktuellen Methangas-Volumenstroms (Vᵢₛₜ) mit einem vorgegebenen Methangas-Volumenstrom (Vₛₒₗₗ) statt.

Weiterhin ist nicht nur der aerobe Aufschluss der Cellulosen und Hemicellulosen zu Zuckern während der belüfteten Intervalle für die maximal zu erreichende Methanerzeugung von Bedeutung sondern auch die Menge des im ersten Abbauschritt gebildeten Zuckers, die im darauf folgenden unbelüfteten anaeroben Intervall zu Ethanol verstoffwechselt wird, aus der dann im nächsten aeroben Intervall Essigsäure gebildet wird. Ein Ungleichgewicht der aeroben und anaeroben Intervalle führt bei zu hohem Sauerstoffeintrag und zu langen belüfteten Intervallen zur Verstoffwechslung des gebildeten Zuckers durch aerobe Bakterien zu Kohlendioxid und Wasser, wodurch der Anteil des Zuckers für die Methanbildung im Fermenter verloren geht. Die Folge ist eine verringerte Effizienz der Methanbildung des Gesamtprozesses. Bei zu geringer Sauerstoffzufuhr und zu kurzen belüfteten Intervallen setzt in den anaeroben Intervallen eine verstärkte anaerobe Hydrolyse der Kohlenwasserstoffe statt, bei der aufgrund des unvollständigen Aufschlusses vorwiegend längerkettige Gärsäuren (vor allem Propionsäure, Buttersäure, Valeriansäure und Capronsäure) gebildet werden. Diese gelangen unverändert in den Fermenter und werden durch den weniger effizienten Stoffwechselweg von acidogenen Bakterien zu Wasserstoff und Kohlendioxid aufgeschlossen, die durch Methan bildende Bakterien ebenfalls zu Biogas vergoren werden. In diesem Fall sinkt ebenfalls die Effizienz der Methanbildung. Aus den Gaskonzentrationen in der Abluft, insbesondere der Sauerstoff- und Kohlendioxidkonzentration sowie den in der Maischeflüssigkeit enthaltenen Proportionen der einzelnen Gärsäuren, dem Gärsäurespektrum, als Stellgrößen werden über einen implementierten Algorithmus die Belüftungsmenge und die Dauer der belüfteten und unbelüfteten Intervalle abgeleitet und damit Maischeprozess gesteuert.

In der Gesamtschau werden also sowohl die Flüssigkeitsmenge als auch die Substratmenge und die Belüftung über geeignete Leitgrößen gesteuert. Durch die kombinierte Steuerung der Flüssigkeitsaufnahme über die Stromaufnahme als auch der Substratzufuhr über den Methangasvolumenstrom kann die Zusammensetzung gemäß ihren strukturviskosen Eigenschaften passgenau eingestellt werden. Die Steuerung der Belüftung durch die Abluftwerte und das Gärsäurespektrum sichert einen optimalen biologischen Aufschluss der Substrate in der Turbomaische.

Ferner wird durch das gewählte Höhe zu Durchmesserverhältnis von wenigstens 1 : 3, bevorzugt größer als 1,6: 1, sowohl der benötigte Investitionsaufwand als auch der Aufwand an Rührenergie deutlich reduziert. Weiterhin ist der Weg der aufsteigenden Luft durch den Behälter wesentlich verlängert. Dadurch wird bei gleicher Luftmenge ein größerer Anteil des Sauerstoffs der Luft in der Flüssigkeit gelöst. Die benötigte Luftmenge wird reduziert und damit auch der Aufwand für die Belüftung des Behälters.

Üblicherweise umfasst Biogas sowohl Methan also auch CO₂, was bei der anaeroben Fermentierung von Essigsäure oder Kohlensäure und Wasserstoff entsteht.

Der Methangasvolumenstrom entspricht dem partiellen Volumengasstrom des Methans in Bezug auf den gesamten Biogasvolumenstrom, also dem Gesamtbiogasvolumengasstrom multipliziert mit dem Anteil an Methan im gesamten Biogas.

Dem Fachmann ist bekannt, den gesamten Biogasvolumenstrom zu messen, als auch den relativen Methangehalt zu bestimmen, woraus sich wiederum der Methangasvolumenstrom ergibt.

Im Zusammenhang mit der vorliegenden Erfindung wird die viskose Flüssigkeit in der Turbomaische, umfassend die biologischen Substrate, als Maische bezeichnet.

Die beanspruchte Anlage dient vorzugsweise modular zur Vorbehandlung von Substraten. Die erzeugte Essigsäure wird nachfolgend in einem Fermenter zu Biogas umgewandelt. Daher findet die Messung des Volumenstroms des Biogases im Fermenter statt. Die beschriebene Anlage ist damit dahingehend ausgebildet, ein Eingangssignal betreffend eines aktuellen Methangas-Volumenstroms (Vᵢₛₜ) zu verarbeiten und ein Ausgangsignal zur Steuerung an die Turbomaische zu liefern. Der Biogasfermenter und die beanspruchte Anlage zur Vorbehandlung von Substraten wirken somit modular und aufeinander abgestimmt zusammen.

Gemäß einer weiteren Ausführungsform wird die Anlage beschrieben, wobei die Anlage zum semi-kontinuierlichem Betrieb ausgebildet ist.

Gemäß einer bevorzugten Ausführungsform wird die Anlage beschrieben, wobei der Behälter ein Höhe zu Durchmesser -Verhältnis von größer 1 : 3 aufweist.

Gemäß einer bevorzugten Ausführungsform weist die Anlage ferner auf:
einen Störstoffabscheider;
eine Nasszerkleinerung;
eine Maischepumpe; und
eine Umpumpleitung. Dabei ist die Anlage ausgebildet, Flüssigkeit aus dem Behälter zum Zerkleinern langer Faserstoffe zu entnehmen und über den Störstoffabscheider, die Nasszerkleinerung und die Maischepumpe durch die Umpumpleitung in den Behälter zurückzufördern.

Gemäß einer weiteren bevorzugten Ausführungsform umfasst die Anlage ferner:
eine Einrichtung zum Mischen fester Einsatzstoffe mit der benötigten Flüssigkeitsmenge;
einen Störstoffabscheider,
eine Nasszerkleinerung;
eine Substratpumpe, und
ein Feststoffdosierer, wobei die Anlage dahingehend ausgebildet ist, dass
die Beschickung des Behälters mit festem Substrat aus dem Feststoffdosierer über die Einrichtung zum Mischen fester Einsatzstoffe mit der benötigten Flüssigkeitsmenge, gefolgt von der Störstoffabscheidung, und der Nasszerkleinerung und der Substratpumpe erfolgt.
Anders als im Falle der zuvor beschriebenen Ausführungsform, bei der die Anlage ausgebildet, Flüssigkeit aus dem Behälter zum Zerkleinern langer Faserstoffe zu entnehmen und über den Störstoffabscheider, die Nasszerkleinerung und die Maischepumpe durch die Umpumpleitung in den Behälter zurückzufördern, dient die vorliegende Ausführungsform dazu, den Behälter zu beschicken. Allerdings können beide Ausführungsformen sinngemäß miteinander kombiniert werden.

Gemäß einer bevorzugten Ausführungsform umfasst die Anlage ferner:
ein Störstoffaustrag, der im Behälter angeordnet ist,
eine Horizontalschnecke, die in einer Mulde geführt wird;
eine außerhalb des Behälters befindlichen Schrägschnecke;
wobei der Störstoffaustrag ausgebildet ist, mittels der in der Nähe des Behälterbodens liegenden Horizontalschnecke, und der Schrägschnecke, eine Säuberung des Behälterbodens zu ermöglichen.

Alternativ zum oben beschriebenen Störstoffaustrag, kann die Säuberung des Behälters, also der Turbomaische, dahingehend erfolgen, dass die Störstoffe nach Entleerung vom Behälterboden abgesaugt werden.

Gemäß einer bevorzugten Ausführungsform wird die Anlage beschrieben, wobei aus einem Zusatzstoffbehälter flüssige Zusatzstoffe mittels einer Zusatzstoffpumpe bei Bedarf in den Behälter gefördert werden können.

Gemäß einer bevorzugten Ausführungsform umfasst die Anlage ferner:
eine Beschickungsöffnung für Einsatzstoffe, wobei
die Beschickungsöffnung dahingehend ausgebildet ist, dass diese während der Beschickung im Kontakt mit der offenen Atmosphäre steht, und zwischen den Beschickungen verschlossen ist.

Gemäß einer bevorzugten Ausführungsform wird die Anlage beschrieben, wobei die Anlage ferner ein Gebläse umfasst, wobei das Gebläse ausgebildet ist, Abluft aus dem Behälter abzuziehen und ferner vorzugsweise über ein Abluftfilter zu reinigen.

Gemäß einer bevorzugten Ausführungsform wird die Anlage beschrieben, wobei die zuzuführende Menge an Flüssigkeit aus dem Vergleich der aktuellen Stromaufnahme (Iᵢₛₜ) des Rührwerks mit der vorgegebenen Stromaufnahme (Iₛₒₗₗ) des Rührwerks dazu dient, die notwendige Temperatur in dem Behälter mittels Heizflüssigkeit und/oder Kühlflüssigkeit einzustellen.

Damit dient die Flüssigkeitszufuhr nicht nur der Regulation der Viskosität, sondern kann ferner auch dazu dienen, die Temperatur in der Maische zu regulieren.

Gemäß einer bevorzugten Ausführungsform wird die Anlage beschrieben, diese ferner wenigstens eine Belüftungseinrichtung umfasst. Bei Stroheinsatz wird durch in der Nähe der Behälterwand angebrachte wenigstens eine Belüftungseinrichtung der Bildung von Schwimmschichten an der Behälterwand entgegenwirkt, indem sie die aufschwimmenden Strohpartikel in Richtung Rührer schieben, die so vom Rührwerk erfasst werden können.

Gemäß einer bevorzugten Ausführungsform wird die Anlage beschrieben, wobei der Behälter ein Höhe zu Durchmesser-Verhältnis von wenigstens 1:1, bevorzugt größer 1,6 : 1 aufweist und nur ein vertikales Großflügel-Propellerrührwerk aufweist. Bei dieser Ausführungsform handelt es sich um eine Alternative für die Anlagengestaltung entsprechend dem angegebenen Verhältnis.

Wiederum wird gemäß dieser Ausführungsform auf das wichtige Höhe zu Durchmesser-Verhältnis abgestellt.

In dieser Bauform wird sowohl der benötigte Investitionsaufwand als auch der Aufwand an Rührenergie deutlich reduziert. Weiterhin ist der Weg der aufsteigenden Luft durch den Behälter wesentlich verlängert. Dadurch wird bei gleicher Luftmenge ein größerer Anteil des Sauerstoffs der Luft in der Flüssigkeit gelöst. Die benötigte Luftmenge wird reduziert und damit auch der Aufwand für die Belüftung des Behälters.

Gemäß einer bevorzugten Ausführungsform wird die Anlage beschrieben, wobei der Behälter ein Höhe zu Durchmesser-Verhältnis von kleiner als 1, : 1 aufweist und ein zweites, schräges Großflügel-Propellerrührwerk eingesetzt ist. Bei dieser Ausführungsform handelt es sich um eine andere Alternative für die Anlagengestaltung entsprechend dem angegebenen geringeren Verhältnis von Höhe zu Durchmesser. Wiederum wird gemäß dieser Ausführungsform auf das wichtige Höhe zu Durchmesser-Verhältnis abgestellt.

Bei sehr breiten Behältern ist das zum Einmischen der festen Substrate benötigte Rührwerk in seinem Durchmesser zu klein, um die notwendige radiale und vertikale Durchmischung des gesamten Behälters und damit sowohl die gleichmäßige Verteilung der festen Substrate als auch der eingespeisten Luft zu gewährleisten. Daher ist es notwendig, ein zweites oder mehrere vorzugsweise schräge Rührwerke mit ebenfalls mindestens zwei Rührstufen in den Behälter einzubringen, der die vollständige Durchmischung des Behälters sichert.

Gemäß einer bevorzugten Ausführungsform wird die Anlage beschrieben, wobei der Behälter bei stark jahreszeitlich flexibilisierter Biogasproduktion mit mindestens drei Propellerstufen ausgerüstet ist, um bei geringer Gasproduktion, z.B. im Sommer, mit geringerem Füllvolumen effektiv betrieben und damit Rührenergie gespart wird.

Gemäß einer bevorzugten Ausführungsform wird die Anlage beschrieben, wobei als Belüftungsorgan Membran-Belüfter genutzt werden, die auf das Belüftungsrohr (8) montiert werden und auswechselbar sind.

Gemäß einer bevorzugten Ausführungsform wird die Anlage beschrieben, wobei das Höhe zu Durchmesser-Verhältnis kleiner als 1 : 2 gewählt wird und neben dem äußeren Belüftungsring ein zweiter oder gegebenenfalls dritter innerer Belüftungsring angeordnet ist, um eine gleichmäßige Luftverteilung im Behälter zu gewährleisten. Wiederum wird gemäß dieser Ausführungsform auf das wichtige Höhe zu Durchmesser-Verhältnis abgestellt.

Gemäß einer bevorzugten Ausführungsform wird die Anlage beschrieben, wobei der Behälter mit einer Revisionsöffnung versehen ist, durch die Wartungsarbeiten an Belüftern und Rührer durchgeführt werden können sowie grobe Störstoffe bei Bedarf entfernt werden können.

Gemäß einem weiteren Aspekt wird eine Biogasanlage beschrieben, umfassend:
eine Anlage zur Vorbehandlung von festen Substraten wie vorstehend beschrieben;
einen Fermenter, aufweisend ein Messgerät zur Erfassung des aktuellen Biogas-Volumenstroms (Vist).

Gemäß einem weiteren Aspekt wird ein Verfahren zur Erzeugung von Biogas aus festen Substraten beschrieben. Die erfindungsgemäße Biogasanlage dient der Ausführung dieses Verfahrens.

Damit wird insbesondere ein Verfahren zur Gewinnung von Biogas beschrieben, umfassend die Schritte:
a) Bereitstellung einer Biogasanlage
b) Erfassen eines aktuellen Biogas-Volumenstroms (Vᵢₛₜ) und
c) Erfassen einer aktuellen Stromaufnahme (Iᵢₛₜ) des Rührwerks;
d) Erfassen der Temperatur;
e) Erfassen der Abluftzusammensetzung und des Säurespektrums in der Turbomaische
f) Vergleichen des aktuellen Biogas-Volumenstroms (Vᵢₛₜ) mit einem vorgegebenen Sollwert für den Biogas-Volumenstroms (Vₛₒₗₗ), sowie
g) Vergleichen der aktuellen Stromaufnahme des Rührwerks (Iᵢₛₜ) mit einem vorgegebenen Sollwert für die Stromaufnahme des Rührwerks (Iₛₒₗₗ); und
h) Bestimmung und gegebenenfalls Zugabe einer zuzuführenden Menge an festen Substraten und zuzuführenden Menge an Flüssigkeit anhand einer Abweichung der Ist-Werte von den SollWerten auf Basis von in der Steuerungseinheit hinterlegten Kennlinien;
i) Bestimmung und Regelung der Temperatur durch Heizen oder Kühlen auf einen vorgegebenen Wert.
j) Steuerung der zugeführten Luftmenge und der Dauer der belüfteten und unbelüfteten Intervalle durch einen vorgegebenen Algorithmus aus der Zusammensetzung der Abluft, insbesondere dem Sauerstoff- und Kohlendioxidgehalt, sowie dem Säurespektrum in der Turbomaische.

Gemäß einer bevorzugten Ausführungsform sind Stellgrößen für die Anlage:
Die Viskosität in der Turbomaische, die durch die Stromaufnahme des Rührers bei einer gegebenen Rührerdrehzahl repräsentiert wird;
Die Gaskonzentrationen, insbesondere von Sauerstoff, Kohlendioxid, Methan und Wasserstoff, in der Abluft im Inneren der Turbomaische;
Die in der Biogasanlage aus den zugeführten Einsatzstoffen erzeugte Methanmenge.

Die Stellgröße ist die Ausgangsgröße (die Stellung) des in der Steuerungs- und in der Regelungstechnik verwendeten Stellglieds, mit dessen Hilfe ein gezielter Eingriff in die Steuerbeziehungsweise Regelstrecke erfolgt. Wie oben ausgeführt, stellt insbesondere die Viskosität der Zusammensetzung eine wichtige Stellgröße dar.

Neben der Stromaufnahme des Rührers und dem Methangasvolumenstrom sind weitere Messgrößen für die Anlage: die Rührerdrehzahl, und/oder die Abluftzusammensetzung der Turbomaische und/oder die Biogasmenge und die Biogaszusammensetzung. Als besonders bevorzugt haben sich allerdings der Methangasvolumenstrom und die Stromaufnahme des Rührers erwiesen.

Die Messgröße ist diejenige physikalische Größe in der Messtechnik, der eine Messung gilt. Dabei wird der Begriff sowohl für "Messgröße im allgemeinen Sinn" als auch für "spezielle Messgröße" verwendet. Die relevante Messgröße für die vorliegende Anlage ist die Stromaufnahme des Rührers in Bezug auf die Flüssigkeitszugabe.

Gemäß einer bevorzugten Ausführungsform sind zusätzliche Steuerungsgrößen für die Anlage:
Die Substratzufuhr und/oder
Die Fugatzufuhr und/oder
Die Luftzufuhr und/oder
Die Länge der belüfteten sowie der unbelüfteten Intervalle und/oder
Die Temperatur der Turbomaische

Die Regelung bzw. das Regeln ist ein Vorgang, bei dem fortlaufend eine Größe, die Regelgröße, erfasst, mit einer anderen Größe, der Führungsgröße, verglichen und im Sinne einer Angleichung an die Führungsgröße beeinflusst wird. In diesem Zusammenhang kann beispielsweise die Luftzufuhr gemäß der Konzentration von Sauerstoff und Kohlendioxid in der Abluft der Turbomaische reguliert werden. Je nach Abluftzusammensetzung der Turbomaische wird die Luftzufuhr also nach oben oder nach unten reguliert. Entsprechendes gilt auch für die weiteren oben genannten Parameter. So können beispielsweise die Substratzufuhr und die Luftzufuhr aufeinander abgestimmt geregelt werden, um somit eine optimierte Betriebsführung zu erreichen.

In Bezug auf die Temperaturregelung ist zu beachten, dass in der Turbomaische unter anderem aerobe biologische Prozesse erfolgen, die im Temperaturbereich zwischen 30 °C und 38 °C ablaufen und eine positive Wärmetönung besitzen. Diese Wärmetönung kann die Selbsterwärmung des Behälters um mehrere Grad Celsius bewirken. Andererseits besitzt insbesondere Mist schon nach sehr kurzer Lagerzeit häufig Temperaturen von bis zu 60 °C. Die zugeführte Flüssigkeit zum Anmischen besteht häufig aus abgepresstem Gärrest, der eine Temperatur von bis zu 45 °C aufweisen kann. Das erfordert eine Temperierung des Behälters, der sowohl im Winter geheizt als auch häufig im Sommer abgekühlt werden muss.

Weitere bevorzugte Ausgestaltungen der Erfindung ergeben sich aus den übrigen, in den Unteransprüchen genannten Merkmalen.

Die verschiedenen in dieser Anmeldung genannten Ausführungsformen der Erfindung sind, sofern im Einzelfall nicht anders ausgeführt, mit Vorteil miteinander kombinierbar.

### Kurzbeschreibung der Figuren

Die Erfindung wird nachfolgend in Ausführungsbeispielen anhand der zugehörigen Zeichnungen erläutert. Es zeigen:
- Figur 1: Turbomaische-Anlage mit direkter Feststoffbeschickung
- Figur 2: Turbomaische-Anlage mit Beschickung über vorgeschaltetes fest-flüssig-Mischsystem
- Figur 3: Turbomaische-Behälter
- Figur 3a: Vertikalschnitt frontal
- Figur 3b: Vertikalschnitt seitlich
- Figur 3c: Querschnitt von oben über erster Propellerstufe

### Detaillierte Beschreibung der Erfindung

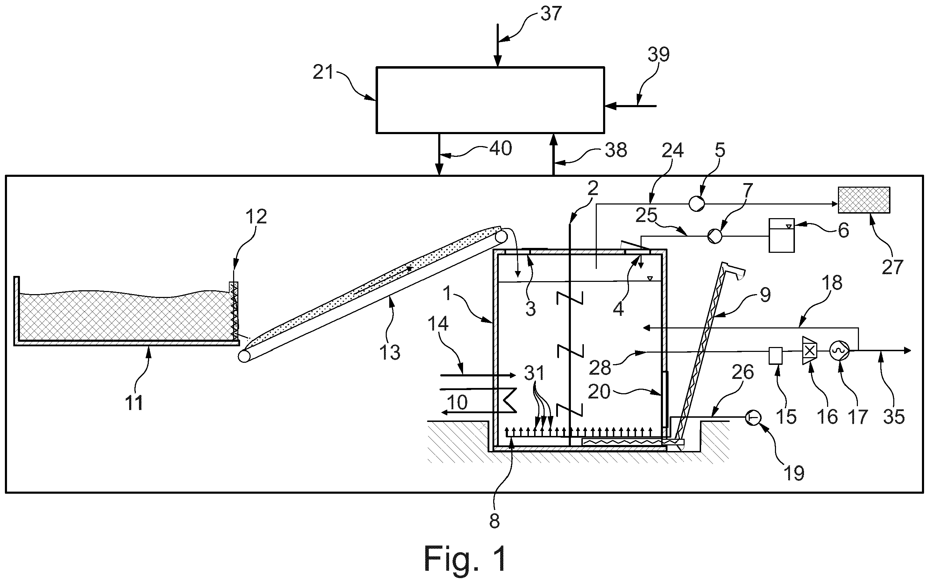

Figur 1 zeigt eine Biogasanlage mit direkter Feststoffbeschickung in die Turbomaische zur Vorbehandlung von Einsatzstoffen in Biogasanlagen. Die Anlage umfasst einen Anmisch- und Hydrolysebehälter 1, welcher als Turbomaische bezeichnet wird.

Der isolierte und temperierbare Behälter ist bevorzugt aus einem gegen Säureeinwirkung beständigem Material, insbesondere aus einem innenseitig beschichteten Beton, emaillierten oder innen beschichteten Stahl oder anderem geeigneten Material dargestellt.

Die Höhe des Behälters relativ zum Durchmesser ist bevorzugt dahingehend gewählt, dass diese mindestens 1 : 3, vorzugsweise 1,6 : 1 beträgt.

Der Behälter 1 weist mindestens ein mindestens zweistufig ausgebildetes Großflügel-Propellerrührwerk 2 zum Einmischen der festen Substrate auf. Der Behälter 1 weist ferner einen nahe der Behälterwand liegenden Belüftungsring 8 mit installierten Belüftungsorganen 31, welche vorzugsweise als Membranbelüfter ausgebildet sind, der durch einen Verdichter 19 über eine Zuluftleitung 26 mit Luft versorgt wird, auf. Am Belüftungsring 8 ist eine Entwässerungsleitung 29, welche zum Entfernen von Kondensatwasser, welches in der Luftleitung anfällt, dient.

Ferner umfasst der Behälter 1 eine verschließbare Beschickungsöffnung 3, die auch als Einwurfsöffnung bezeichnet werden kann, für die Zufuhr der festen Substrate. Die festen Substrate werden dabei durch die verschließbare Beschickungsöffnung, aus einem Feststoffdosierer 11 mit Auflockerungseinrichtung 12 in die Anlage eingeführt. Dies erfolgt über eine Fördereinrichtung 13, insbesondere ein Förderband oder Förderschnecke, die die Einsatzstoffe von oben in die Turbomaische einbringt.

Weiter enthält der Behälter eine verschließbare Inspektionsöffnung 4, die es erlaubt, die Flüssigkeitsoberfläche zu beobachten, um das Einmischen der festen Substrate und die Blasenverteilung visuell zu beurteilen sowie bei Bedarf Proben zu nehmen oder Zusatzstoffe zugegeben zu können.

Ferner werden eine Zuleitung für Flüssigkeiten zum Anmischen der Feststoffe 14, als auch eine Vorrichtung zum Temperieren der Turbomaische 10, die es erlaubt, den Behälterinhalt im Winter zu heizen und im Sommer zu kühlen, verwendet. Eine Revisionsöffnung 20 ist im Behälter vorgesehen.

Das vorbehandelte Maischematerial wird durch die Maischeentnahme 28 über die Maischeleitung 35 in den Methanfermenter gefördert. Die Maischeleitung enthält eine Pumpe 17, die in Lage ist dickflüssige Stoffe zu transportieren und eine Nasszerkleinerungseinrichtung 16, die dem Zerkleinern langfaserigen Materials dient.

Ein Steinfang 15 ist der Nasszerkleinerungseinrichtung vorgeschaltet. Zeitweise wird Flüssigkeit aus der Turbomaische 1 zum Zerkleinern langer Faserstoffe entnommen und über Störstoffabscheider 15, Nasszerkleinerung 16 und die Maischepumpe 17 durch eine Umpumpleitung 18 in den Turbomaischebehälter 1 zurückgefördert. Ein Störstoffaustrag 9 ist im Behälter installiert. Aus einem Zusatzstoffbehälter 6 werden flüssige Zusatzstoffe mittels einer Zusatzstoffpumpe 7 bei Bedarf über die Zusatzstoffleitung 25 in die Turbomaische gefördert. Die Abluft der Turbomaische wird mittels des Abluftgebläses 5 über die Abluftleitung 24 in die Abluftbehandlung 27 gefördert. Mittels einer Steuerungseinheit 21 werden die vorgegebenen Stellgrößen 37 (Temperatur, Methanvolumenstrom, Viskosität in der Turbomaische und Säurespektrum) in der Turbomaische aus den Messgrößen der Turbomaische 38 (Stromaufnahme des Rührers, Rührerdrehzahl und Abluftzusammensetzung) sowie die Messgrößen 39 des Biogasfermenters (Biogasvolumenstrom und Biogaszusammensetzung, insbesondere Methangehalt) durch die Regelgrößen 40 (Substratzufuhr, Flüssigkeitszufuhr, Luftzufuhr, Belüftungsintervall und Heiz-/ Kühlmittelzufuhr) geregelt. Ferner ist der Behälter mit einer Revisionsöffnung 20 versehen, wodurch Wartungsarbeiten an Belüftern und Rührer durchgeführt werden können, sowie grobe Störstoffe bei Bedarf entfernt werden können. Ferner enthält die Turbomaische einen Störstoffaustrag bestehend aus Mulde 32, Horizontalschnecke 33 und Schrägschnecke 34.

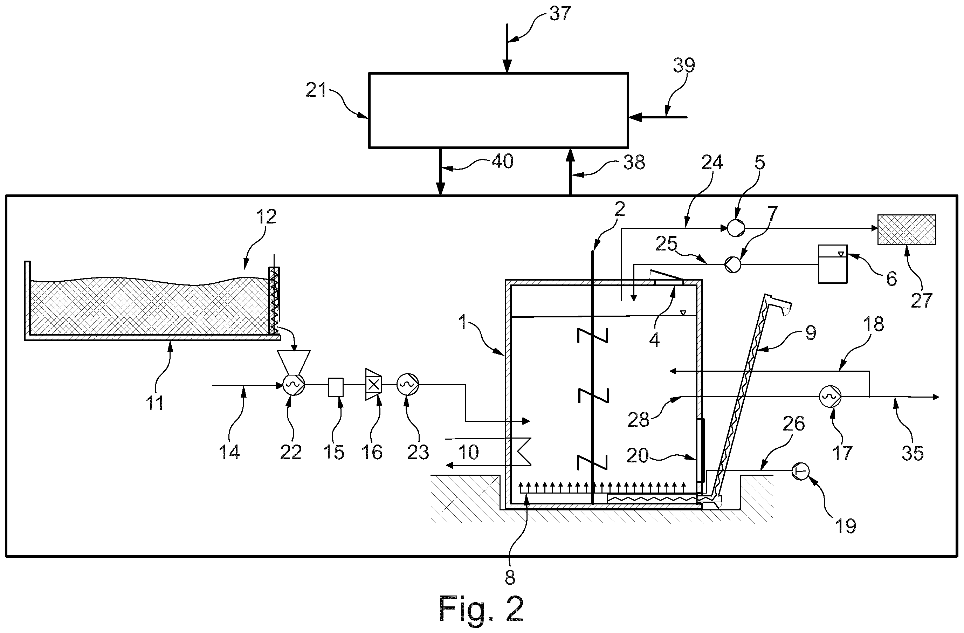

Figur 2 zeigt eine Biogasanlage mit Beschickung der Einsatzstoffmischung über ein vorgeschaltetes fest-flüssig Mischsystem. Während in der Ausführungsform gemäß Figur 1 noch während der Hydrolysereaktion zeitweise Flüssigkeit aus der Turbomaische zum Zerkleinern langer Faserstoffe entnommen und über Störstoffabscheider 15, Nasszerkleinerung 16 und Maischepumpe 17 durch eine Umpumpleitung 18 in den Turbomaischebehälter 1 zurückgefördert wird, findet dies in gemäß der Ausführungsform nach Figur 2 durch ein vorgeschaltetes System aus Störstoffabscheider 15 und Nasszerkleinerung 16 statt.

In Figur 2 ist also die Anlage dahingehend ausgebildet, dass die Beschickung der Turbomaische durch eine geeignete Einrichtung zum Mischen fester Einsatzstoffe 22 mit der benötigten Flüssigkeitsmenge, gefolgt von einer Störstoffabscheidung 15, einer Nasszerkleinerung 16 und einer Substratpumpe 23 erfolgt. In beiden gezeigten Ausführungsforme ist die Anlage bevorzugt dahingehend ausgebildet, dass die zur Flüssigbeschickung genutzten Komponenten 22, 15, 16 und 23 in beliebiger Weise in einer oder mehreren Maschinen kombiniert sind.

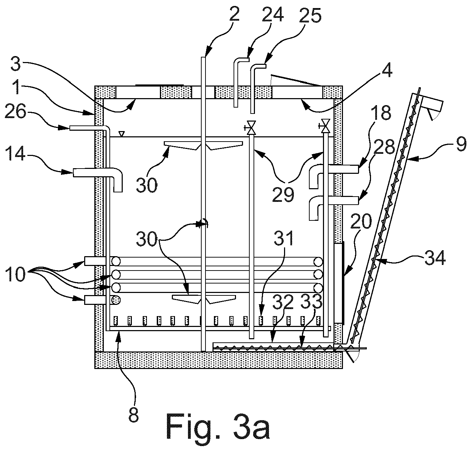

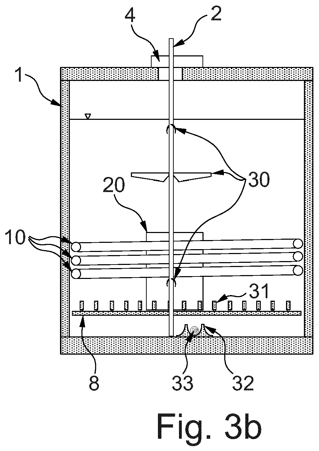

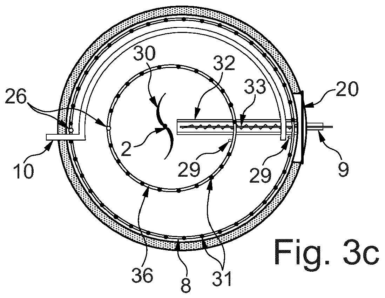

Figur 3a zeigt den Biogasreaktor im Vertikalschnitt, zentral, Frontansicht, während Figur 3b Biogasreaktor im Vertikalschnitt, zentral, Seitenansicht zeigt. Figur 3c zeigt den Biogasreaktor im Querschnitt unten über erster Propellerstufe.

Die Figur 3a zeigt ferner den Störstoffaustrag 9 im Detail, der im Behälter installiert ist, der mittels einer in der Nähe des Behälterbodens liegenden Horizontalschnecke 33, die in einer Mulde 32 geführt wird, und einer außerhalb des Behälters befindlichen Schrägschnecke 34 bei hohem Sandgehalt der Einsatzstoffe eine regelmäßige Säuberung des Behälterbodens ermöglicht.

Die Anlage ist ferner bevorzugt dahingehend ausgebildet, dass der Behälter bei stark jahreszeitlich flexibilisierter Biogasproduktion mit mindestens drei Propellerstufen 30 ausgerüstet ist, um bei geringer Gasproduktion, z.B. im Sommer, mit geringerem Füllvolumen effektiv betrieben und damit Rührenergie gespart wird. Dies wird insbesondere aus Figur 2a und Figur 2b deutlich.

Ferner wird gezeigt, dass als Belüftungsorgane vorzugsweise Membran-Belüfter 31 genutzt werden, die auf das Belüftungsrohr 8 montiert werden und auswechselbar sind, wie aus Figur 3a, Figur 3b und Figure 3c deutlich wird. Insbesondere Figur 3c zeigt dabei wie die Belüfter im Querschnitt auf dem Belüftungsrohr angeordnet sind.

Die Turbomaische kann bevorzugt dahingehend ausgestaltet sein, dass bei einem Höhe zu Durchmesser-Verhältnis kleiner als 1 : 1 neben dem äußeren Belüftungsring ein zweiter innerer Belüftungsring 36 oder auch ein weiterer innerer Belüftungsring angeordnet ist, um eine gleichmäßige Luftverteilung im Behälter zu gewährleisten.

Ferner weist die Anlage eine Abluftleitung 24, eine Zusatzstoffleitung 25, eine Zuluftleitung 26, eine Heiz-/ Kühlleitung 10 und eine Maischeentnahme 28 auf.

### Bezugszeichenliste

- 1: Turbomaische Behälter
- 2: Rührer
- 3: Einwurfsöffnung
- 4: Inspektionsöffnung
- 5: Abluftgebläse
- 6: Zusatzstoffbehälter
- 7: Zusatzstoffpumpe
- 8: Belüftungsring mit Belüftern
- 9: Störstoffaustrag
- 10: Heiz-/ Kühlleitung
- 11: Feststoffdosierer
- 12: Auflockerung
- 13: Fördereinrichtung
- 14: Flüssigkeitszufuhr
- 15: Störstoffabscheider
- 16: Nasszerkleinerung
- 17: Maischepumpe
- 18: Umpumpleitung
- 19: Verdichter
- 20: Revisionsöffnung
- 21: Steuerungseinheit
- 22: Fest-Flüssig-Mischer
- 23: Substratpumpe
- 24: Abluftleitung
- 25: Zusatzstoffleitung
- 26: Zuluftleitung
- 27: Abluftreinigung
- 28: Maischeentnahme
- 29: Entwässerungsleitung mit Ventil
- 30: Propeller
- 31: Membran-Belüfter
- 32: Mulde zum Störstoffaustrag
- 33: Horizontalschnecke
- 34: Schrägschnecke
- 35: Maischeleitung
- 36: innerer Belüftungsring
- 37: Stellgrößen:
Temperatur;
Methanvolumenstrom Viskosität;
Gärsäurespektrum;
- 38: Messgrößen Turbomaische:
Stromaufnahme des Rührers;
Rührerdrehzahl;
Abluftzusammensetzung;
- 39: Messgrößen Biogasfermenter:
Biogasvolumenstrom;
Biogaszusammensetzung;
- 40: Regelgrößen:
Substratzufuhr;
Flüssigkeitszufuhr;
Luftzufuhr;
Belüftungsintervall;
Heiz-/ Kühlmittelzufuhr;

## Patentansprüche

1. Anlage zur Vorbehandlung von festen Substraten in Biogasanlagen, umfassend:
einen Behälter (1), dessen Höhe zu Durchmesser-Verhältnis mindestens 1 : 3 beträgt, ein mindestens zweistufiges Großflügel-Propellerrührwerk (2) zum Einmischen der festen Substrate; und
einen Belüftungsring (9) umfassend wenigstens 1 Belüftungsorgan (31), welches durch einen Verdichter (19) mit Luft versorgt wird;
eine Heiz-/ Kühlleitung (10) sowie eine Steuerungseinheit (21), die ausgebildet ist:
eine zuzuführende Menge an Flüssigkeit aus dem Vergleich einer aktuellen Stromaufnahme (Iᵢₛₜ) des Rührwerks mit einer vorgegebenen Stromaufnahme (Iₛₒₗₗ) des Rührwerks zu steuern; und
die Temperatur in der Turbomaische zu regeln; und
die zuzuführende Luftmenge sowie die Dauer der belüfteten Intervalle und unbelüfteten Intervalle aus der Bewertung der Gaskonzentrationen in einer Abluft, insbesondere von Sauerstoff und Kohlendioxid, und/oder dem Gärsäurespektrum in der Maischeflüssigkeit zu steuern.

2. Anlage nach Anspruch 1, wobei die Steuerungseinheit dahingehend ausgebildet ist, eine zuzuführende Menge an festen Substraten aus dem Vergleich eines aktuellen Methangas-Volumenstroms (Vᵢₛₜ) mit einem vorgegebenen Methangas-Volumenstrom (Vₛₒₗₗ) zu steuern;

3. Anlage nach Anspruch 1, wobei die Anlage ferner aufweist:
einen Störstoffabscheider (15);
eine Nasszerkleinerung (16);
eine Maischepumpe (17); und
eine Umpumpleitung (18);
und wobei die Anlage ausgebildet ist, Flüssigkeit aus dem Behälter (1) zum Zerkleinern langer Faserstoffe zu entnehmen und über den Störstoffabscheider (15), die Nasszerkleinerung (16) und die Maischepumpe (17) durch die Umpumpleitung (18) in den Behälter (1) zurückzufördern.

4. Anlage gemäß einem der vorhergehenden Ansprüche, wobei die Anlage ferner umfasst:
ein Störstoffaustrag (9); der im Behälter (1) angeordnet ist,
eine Horizontalschnecke (33), die in einer Mulde (32) geführt wird;
eine außerhalb des Behälters (1) befindlichen Schrägschnecke (34);
wobei der Störstoffaustrag (9) ausgebildet ist, mittels der in der Nähe des Behälterbodens liegenden Horizontalschnecke (33), und der Schrägschnecke (34), eine Säuberung des Behälterbodens zu ermöglichen.

5. Anlage gemäß einem der vorhergehenden Ansprüche, wobei die Anlage ferner umfasst:
eine Einrichtung zum Einmischen fester Einsatzstoffe (22) in Flüssigkeit;
einen Störstoffabscheider (15);
eine Nasszerkleinerung (16);
eine Substratpumpe (23), und
ein Feststoffdosierer (11),
wobei die Anlage dahingehend ausgebildet ist, dass
die Beschickung des Behälters mit festem Substrat aus dem Feststoffdosierer (11) über die Einrichtung zum Mischen fester Einsatzstoffe (22) mit der benötigten Flüssigkeitsmenge, gefolgt von der Störstoffabscheidung (15), und der Nasszerkleinerung (16) und der Substratpumpe (23) erfolgt.

6. Anlage gemäß einem der vorhergehenden Ansprüche, wobei die Anlage ferner umfasst:
ein Gebläse (5), wobei das Gebläse ausgebildet ist, Abluft aus dem Behälter (1) abzuziehen und ferner vorzugsweise über eine Abluftreinigung (27) zu reinigen.

7. Anlage gemäß einem der vorhergehenden Ansprüche, wobei die zuzuführende Menge an Flüssigkeit aus dem Vergleich der aktuellen Stromaufnahme (Iᵢₛₜ) des Rührwerks mit der vorgegebenen Stromaufnahme (Iₛₒₗₗ) des Rührwerks dazu dient, die notwendige Temperatur in dem Behälter (1) mittels Heizflüssigkeit und/oder Kühlflüssigkeit einzustellen.

8. Anlage gemäß einem der vorhergehenden Ansprüche, wobei der Behälter ein Höhe zu Durchmesser-Verhältnis von größer 1 : 1 aufweist; bevorzugt größer 1,6 : 1, und das Großflügel-Propellerrührwerk (2) als ein einziges vertikales Großflügel-Propellerrührwerk ausgebildet ist.

9. Anlage gemäß einem der vorhergehenden Ansprüche, wobei der Behälter (1) mit mindestens drei Propellerstufen (30) ausgerüstet ist.

10. Anlage gemäß einem der vorhergehenden Ansprüche, wobei
das Höhe zu Durchmesser-Verhältnis kleiner als 1 : 3 gewählt wird; und
die Anlage zusätzlich zu dem äußeren Belüftungsring einen zweiten inneren Belüftungsring aufweist und vorzugsweise einen dritten Belüftungsring aufweist.

11. Anlage gemäß einem der vorhergehenden Ansprüche, wobei die Anlage ferner aufweist, einen oder mehrere ausgewählt aus:
eine Substratzuführung für feste Substrate bestehend aus Feststoffdosierer (11), Auflockerung (12) und Fördereinrichtung (13);
eine Einwurfsöffnung (3), wobei die Einwurfsöffnung (3) bevorzugt dahingehend ausgebildet ist, dass diese während der Beschickung im Kontakt mit der offenen Atmosphäre stehen kann, und zwischen den Beschickungen verschließbar ist;
eine Inpektionsöffnung (4);
eine Flüssigkeitszufuhr (14);
eine Revisionsöffnung (20);
eine Maischeentnahme (28) und eine Maischeleitung (35).
eine Beschickungsöffnung (3) für Einsatzstoffe.

12. Anlage gemäß einem der vorhergehenden Ansprüche, wobei
der Behälter ein Höhe zu Durchmesser-Verhältnis von kleiner als 1 : 1 aufweist und ferner ein zweites oder mehrere, schräge Großflügel-Propellerrührwerke (2) umfasst.

13. Biogasanlage umfassend
eine Anlage zur Vorbehandlung von festen Substraten gemäß einem der vorhergehenden Ansprüche;
einen Fermenter.

14. Verfahren zur Gewinnung von Biogas, umfassend die Schritte:
a) Bereitstellung einer Anlage nach Anspruch 13;
b) Erfassen einer aktuellen Stromaufnahme (Iᵢₛₜ) des Rührwerks;
c) Erfassen der Temperatur (Tᵢₛₜ) der Maische;
d) Erfassen des Gasgehaltes (C_{Ist}) der Abluft.
e) Vergleichen der aktuellen Stromaufnahme des Rührwerks (Iᵢₛₜ) mit einem vorgegebenen Sollwert für die Stromaufnahme des Rührwerks (Iₛₒₗₗ);
f) Vergleichen der Temperatur (Tᵢₛₜ) mit dem einem vorgegebenen Sollwert (Tₛₒₗₗ);
g) Vergleichen des Sauerstoffgehaltes (C_{Ist}) mit einem vorgegebenen Sollwert (Cₛₒₗₗ)
